# EUROPEAN PATENT SPECIFICATION

(11) **EP 2 241 552 B1**
(45) Date of publication and mention of the grant of the patent: **14.11.2018**
(21) Application number: 09708203.6
(22) Date of filing: 05.02.2009
(51) Int. Cl.: C07D 201/04, B01J 31/02, C07D 201/02, C07D 225/02, C07D 251/26, C07B 61/00

(54) **PRODUCTION METHOD AND BECKMANN REARRANGEMENT CATALYST FOR PRODUCING A CYCLIC LACTAM COMPOUND**
PRODUKTIONSVERFAHREN UND BECKMANN-NEUORDNUNGSKATALYSATOR ZUR HERSTELLUNG EINER ZYKLISCHEN LACTAM-VERBINDUNG
PROCÉDÉ DE FABRICATION ET CATALYSEUR DE TRANSPOSITION DE BECKMANN POUR PRODUIRE UN COMPOSÉ LACTAME CYCLIQUE

(30) Priority: 08.02.2008 JP 2008029480
(43) Date of publication of application: 20.10.2010
(73) Proprietor: National University Corporation Nagoya University, Aichi 464-8601 (JP); Ube Industries, Ltd., Ube-shi, Yamaguchi 755-8633 (JP)
(72) Inventor: ISHIHARA, Kazuaki, Nagoya-shi Aichi 464-8601 (JP); FUKUDA, Yasuhisa, Ube-shi Yamaguchi 755-8633 (JP); SUGIMOTO, Tsunemi, Ube-shi Yamaguchi 755-8633 (JP); KUGIMOTO, Junichi, Ube-shi Yamaguchi 755-8633 (JP)
(74) Representative: Cabinet Plasseraud
(86) International application number: PCT/JP2009/051946
(87) International publication number: WO 2009/099134

(56) References cited:
- WO-A1-2007/105482
- WO-A1-2008/078642
- JP-A- 2006 219 470
- GRIGAT, ERNST ET AL: "Cyanic acid esters. XIII. Reaction of cyanic esters with oximes", CHEMISCHE BERICHTE , 99(7), 2361-70 CODEN: CHBEAM; ISSN: 0009-2940, 1966, XP000002658208,
- ARTHUR CHAPMAN: "Studies of the Beckmann Change. Part II. The Kinetics of the Spontaneous Rearragement and Solvent Effects", JOURNAL OF THE CHEMICAL SOCIETY, CHEMICAL SOCIETY, LETCHWORTH; GB, 1 January 1934 (1934-01-01), pages 1550-1555, XP008080723, ISSN: 0368-1769
- FURUYA, Y. ET AL.: 'Cyanuric Chloride as a Mild and Active Beckmann Rearrangement Catalyst' JOURNAL OF THE AMERICAN CHEMICAL SOCIETY vol. 127, no. 32, 23 July 2005, pages 11240 - 11241, XP002441494

## Description

### Technical Field

The present invention relates to a process for producing a lactam compound useful as a starting material for Nylons. In particular, it relates to a process for producing a lactam using a cycloalkylidene-aminooxy-1,3,5-triazine compound.

### Background Art

Non-patent reference 1 and Patent references 1 and 2 have described a process for producing an amide compound by conducting Beckmann rearrangement using an aromatic-containing compound containing heteroatom(s) or carbon atom(s) having electron-withdrawing group(s) and leaving group(s) as a catalyst for a rearrangement reaction of an oxime compound. There is described trichlorotriazine as a compound having particularly excellent catalyst activity. However, trichlorotriazine is highly irritative and may be allergenic. It violently and exothermically reacts with water to give cyanuric acid, which is catalytically inactive. Furthermore, during the reaction, there generates corrosive hydrogen chloride gas. This is definitely disadvantageous in industrially producing an amide compound using trichlorotriazine. For example, water dissolved in the oxime and in a solvent, if used, must be removed. Industrially, an oxime is generally produced by reacting a corresponding ketone with an aqueous solution of hydroxylamine. Therefore, the oxime or the oxime solution (when a solvent is used) contains several thousands ppm of dissolved water. Since such water inactivates trichlorotriazine, a dehydrating apparatus must be used for the oxime or the oxime solution. Trichlorotriazine must be, of course, handled under a dry atmosphere. Furthermore, if cyanuric acid is produced in the rearrangement apparatus, it is poorly dissolved in an organic solvent, leading to precipitation of cyanuric acid, which may cause operation troubles. Generated hydrogen chloride gas requires the use of expensive corrosion-resistant materials in a rearrangement apparatus and its attached pipes. In addition, Non-patent reference 1 and Non-patent reference 2 have described a 1,3,5-triazine compound in which all of positions 2, 4 and 6 are substituted with acetophenone oxime. However, in synthesizing a lactam compound, a byproduct from acetophenone oxime is mixed with a desired lactam to be an undesirable impurity. Furthermore, Non-patent reference 2 has described a 1,3,5-triazine compound in which all of positions 2, 4 and 6 are substituted with cyclohexanone oxime, but Beckmann rearrangement using this compound is unknown.
Non-patent reference 1: J. Amer. Chem. Soc., 127(32), 11240 (2005).
Non-patent reference 2: Chem. Ber., 99(7), 2361(1966).

Patent reference 1: Japanese Laid-open Patent Publication No. 2006-219470.
Patent reference 2: PCT International Publication WO 2007/125002.
Patent reference 3: Japanese Laid-open Patent Publication No. S51-46109.

### Disclosure of the Invention

### Problems to be Solved by the Invention

In view of the problems in the prior art, an objective of the present invention is to provide a catalyst which is highly active, stable and safe, forms less byproducts and exhibits good handling properties for a process for producing a lactam, and a novel production process.

### Means for Solving the Problems

We have found that in a process for producing a lactam compound, the desired lactam compound can be obtained in a good yield by using a triazine compound having substituent(s) of cycloalkanone oxime compound moiety. The present invention relates to a process for producing a lactam compound from a cycloalkanone oxime compound in a reaction process in the presence of:
(a) a cycloalkylidene-aminooxy-1,3,5-triazine compound selected from the group consisting of 2,4-dichloro-6-cyclododecylidene-aminooxy-1,3,5-triazine and 2-chloro-4,6-bis(cyclododecylidene-aminooxy)-1,3,5-triazine as a Beckmann rearrangement catalyst,
(b) a solvent selected from the group consisting of aromatic hydrocarbons and acetonitrile, and
(c) optionally, an acid;
wherein said lactam compound is laurolactam.
The present invention also relates to a process for producing a lactam compound, wherein a cycloalkylidene-aminooxy-1,3,5-triazine compound selected from the group consisting of
2,4-dichloro-6-cyclododecylidene-aminooxy-1,3,5-triazine and 2-chloro-4,6-bis(cyclododecylidene-aminooxy)-1,3,5-triazine is provided as a reaction starting material in a reaction using a solvent selected from the group consisting of aromatic hydrocarbons and acetonitrile, and wherein said lactam compound is laurolactam.
The present invention also relates to a Beckmann rearrangement catalyst selected from the group consisting of
2,4-dichloro-6-cyclododecylidene-aminooxy-1,3,5-triazine and
2-chloro-4,6-bis(cyclododecylidene-aminooxy)-1,3,5-triazine, used in a reaction process to prepare a lactam compound from a cycloalkanone oxime compound in the presence of:
(a) a solvent selected from the group consisting of aromatic hydrocarbons and acetonitrile, and
(b) optionally, an acid;
wherein said lactam compound is laurolactam.
The present invention also relates to
2,4-Dichloro-6-cyclododecylidene-aminooxy-1,3,5-triazine or
2-chloro-4,6-bis(cyclododecylidene-aminooxy)-1,3,5-triazine.

The following items are described.
[1] A process for producing a lactam compound, wherein a cycloalkylidene-aminooxy-1,3,5-triazine compound is provided as a Beckmann rearrangement catalyst and/or a reaction starting material in a reaction process to prepare a lactam compound.
[2] The process as described in [1], wherein the number of the cycloalkylidene-aminooxy group in said cycloalkylidene-aminooxy-1,3,5-triazine compound is 1 to 3 and the number of carbon atoms as ring members in the cycloalkylidene group is 5 to 18.
[3] The process as described in [2], wherein said cycloalkylidene-aminooxy-1,3,5-triazine compound is selected from the group consisting of 2,4-dichloro-6-cyclododecylidene-aminooxy-1,3,5-triazine and 2-chloro-4,6-bis(cyclododecylidene-aminooxy)-1,3,5-triazine.
[4] The process as described in any of [1] to [3], wherein said lactam compound prepared is laurolactam.
[5] The process as described in any of [1] to [4], wherein said cycloalkylidene-aminooxy-1,3,5-triazine compound is provided as a Beckmann rearrangement catalyst and a cycloalkanone oxime compound is provided as a starting material, and wherein a lactam compound is produced from said cycloalkanone oxime compound by Beckmann rearrangement.
[6] The process as described in any of [1] to [4], wherein said cycloalkylidene-aminooxy-1,3,5-triazine compound is provided as a starting material and wherein a lactam compound corresponding to the cycloalkylidene-aminooxy group in said compound is produced.
[7] A Beckmann rearrangement catalyst used in producing a lactam compound from a cycloalkanone oxime compound by Beckmann rearrangement, selected from cycloalkylidene-aminooxy-1,3,5-triazine compounds.
[8] The Beckmann rearrangement catalyst as described in [7], wherein the number of the cycloalkylidene-aminooxy group in said cycloalkylidene-aminooxy-1,3,5-triazine compound is 1 to 3 and the number of carbon atoms as ring members in the cycloalkylidene group is 5 to 18.
[9] The Beckmann rearrangement catalyst as described in [8] selected from the group consisting of
   2,4-dichloro-6-cyclododecylidene-aminooxy-1,3,5-triazine and
   2-chloro-4,6-bis(cyclododecylidene-aminooxy)-1,3,5-triazine.
[10] 2,4-Dichloro-6-cyclododecylidene-aminooxy-1,3,5-triazine or 2-chloro-4,6-bis(cyclododecylidene-aminooxy)-1,3,5-triazine.

### Effect of the Invention

According to the present invention, a lactam compound can be produced in a high yield while minimizing byproducts. Furthermore, a cycloalkylidene-aminooxy-1,3,5-triazine compound used is less irritative than trichlorotriazine, so that a lactam compound can be produced more safely. Since the triazine compound used is less reactive with water and more stable than trichlorotriazine, a lactam compound can be easily produced without using a special apparatus for drying an oxime or a particular dehydrated solvent. Thus, the present invention is particularly suitable for production in an industrial scale.

### Best Mode for Carrying Out the Invention

In the present invention, a cycloalkylidene-aminooxy-1,3,5-triazine compound is provided in a reaction process (whether a batch process or continuous process) as a Beckmann rearrangement catalyst and/or reaction starting material. A cycloalkylidene-aminooxy-1,3,5-triazine compound can be used as a Beckmann rearrangement catalyst and from a cycloalkanone oxime compound synthesized is a corresponding lactam (Embodiment 1, as claimed in claim 1). Alternatively, a cycloalkylidene-aminooxy-1,3,5-triazine compound itself can be used as a direct starting material for synthesizing a lactam corresponding to a cycloalkylidene-aminooxy group (Embodiment 2, as claimed in claim 2). The term, "corresponding lactam" means a lactam produced by Beckmann rearrangement of the cycloalkanone oxime compound in Embodiment 1, and also means a lactam produced by Beckmann rearrangement of a cycloalkanone oxime compound which is derived by addition of H to a cycloalkylidene-aminooxy group in a cycloalkylidene-aminooxy-1,3,5-triazine compound in Embodiment 2. When there is the same correspondence relation, the term, "corresponding cycloalkylidene-aminooxy group" or "corresponding cycloalkanone oxime compound" may be used.
In the present invention, said lactam compound is laurolactam.

### Cycloalkylidene-aminooxy-1,3,5-triazine compound

First, there will be described a cycloalkylidene-aminooxy -1,3,5-triazine compound.

A cycloalkylidene-aminooxy-1,3,5-triazine compound is represented by Formula I. 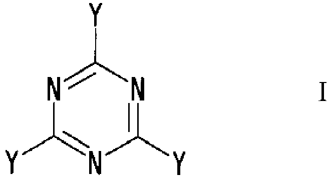 wherein
three Ys, independently of one another, denote cycloalkylidene-aminooxy group represented by Formula II: 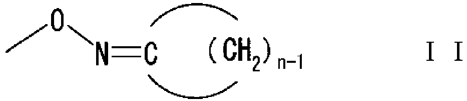 wherein n is the number of ring-member carbon atoms in the cycloalkylidene group,
or
substituent X.

A cycloalkylidene-aminooxy group in a cycloalkylidene-aminooxy-1,3,5-triazine compound preferably has a cycloalkylidene ring with n of 5 to 18 as the number of ring-constituting carbon atoms. Specific examples include cyclopentylidene-aminooxy, cyclohexylidene-aminooxy, cycloheptylidene-aminooxy, cyclooctylidene-aminooxy, cyclononylidene-aminooxy, cyclodecylidene-aminooxy, cycloundecylidene-aminooxy, cyclododecylidene-aminooxy, cyclotridecylidene-aminooxy, cyclotetradecylidene-aminooxy, cyclopentadecylidene-aminooxy, cyclohexadecylidene-aminooxy, cycloheptadecylidene-aminooxy or cyclooctadecylidene-aminooxy. A cycloalkylidene ring may have a substituent which does not inhibit the reaction. Examples include acyclic or cyclic alkyls such as methyl, ethyl and cyclohexyl; acyclic or cyclic alkenyls such as vinyl and cyclohexenyl; aryls such as phenyl; alkoxys such as methoxy; alkoxycarbonyls such as methoxycarbonyl; and halogens such as chloro. It is preferably unsubstituted cycloalkylidene-aminooxy, particularly preferably cyclohexylidene-aminooxy or cyclododecylidene-aminooxy, especially preferably cyclododecylidene-aminooxy.

The number of the cycloalkylidene-aminooxy group in a cycloalkylidene-aminooxy-1,3,5-triazine compound is 1 to 3, preferably 1 or 2. When the number of cycloalkylidene-aminooxy group is 1 or 2, another substituent X on the 1,3,5-triazine ring is preferably halogen such as fluorine, chlorine, bromine and iodine, particularly preferably chlorine.

When there are a plurality of cycloalkylidene-aminooxy groups in a molecule, these cycloalkylidene-aminooxy groups are preferably the same.

Specific examples of the cycloalkylidene-aminooxy-1,3,5-triazine compound may include
2,4-dichloro-6-cyclopentylidene-aminooxy-1,3,5-triazine,
2-chloro-4,6-bis(cyclopentylidene-aminooxy)-1,3,5-triazine,
2,4,6-tris(cyclopentylidene-aminooxy)-1,3,5-triazine,
2,4-dichloro-6-cyclohexylidene-aminooxy-1,3,5-triazine,
2-chloro-4,6-bis(cyclohexylidene-aminooxy)-1,3,5-triazine,
2,4,6-tris(cyclohexylidene-aminooxy)-1,3,5-triazine,
2,4-dichloro-6-cycloheptylidene-aminooxy-1,3,5-triazine,
2-chloro-4,6-bis(cycloheptylidene-aminooxy)-1,3,5-triazine,
2,4,6-tris(cycloheptylidene-aminooxy)-1,3,5-triazine,
2,4-dichloro-6-cyclooctylidene-aminooxy-1,3,5-triazine,
2-chloro-4,6-bis(cyclooctylidene-aminooxy)-1,3,5-triazine,
2,4,6-tris(cyclooctylidene-aminooxy)-1,3,5-triazine,
2,4-dichloro-6-cyclononylidene-aminooxy-1,3,5-triazine,
2-chloro-4,6-bis(cyclononylidene-aminooxy)-1,3,5-triazine,
2,4,6-tris(cyclononylidene-aminooxy)-1,3,5-triazine,
2,4-dichloro-6-cyclodecylidene-aminooxy-1,3,5-triazine,
2-chloro-4,6-bis(cyclodecylidene-aminooxy)-1,3,5-triazine,
2,4,6-tris(cyclodecylidene-aminooxy)-1,3,5-triazine,
2,4-dichloro-6-cycloundecylidene-aminooxy-1,3,5-triazine,
2-chloro-4,6-bis(cycloundecylidene-aminooxy)-1,3,5-triazine,
2,4,6-tris(cycloundecylidene-aminooxy)-1,3,5-triazine,
2,4-dichloro-6-cyclododecylidene-aminooxy-1,3,5-triazine,
2-chloro-4,6-bis(cyclododecylidene-aminooxy)-1,3,5-triazine,
2,4,6-tris(cyclododecylidene-aminooxy)-1,3,5-triazine,
2,4-dichloro-6-cyclotridecylidene-aminooxy-1,3,5-triazine,
2-chloro-4,6-bis(cyclotridecylidene-aminooxy)-1,3,5-triazine,
2,4,6-tris(cyclotridecylidene-aminooxy)-1,3,5-triazine,
2,4-dichloro-6-cyclotetradecylidene-aminooxy-1,3,5-triazine,
2-chloro-4,6-bis(cyclotetradecylidene-aminooxy)-1,3,5-triazine,
2,4,6-tris(cyclotetradecylidene-aminooxy)-1,3,5-triazine,
2,4-dichloro-6-cyclopentadecylidene-aminooxy-1,3,5-triazine,
2-chloro-4,6-bis(cyclopentadecylidene-aminooxy)-1,3,5-triazine,
2,4,6-tris(cyclopentadecylidene-aminooxy)-1,3,5-triazine,
2,4-dichloro-6-cyclohexadecylidene-aminooxy-1,3,5-triazine,
2-chloro-4,6-bis(cyclohexadecylidene-aminooxy)-1,3,5-triazine,
2,4,6-tris(cyclohexadecylidene-aminooxy)-1,3,5-triazine,
2,4-dichloro-6-cycloheptadecylidene-aminooxy-1,3,5-triazine,
2-chloro-4,6-bis(cycloheptadecylidene-aminooxy)-1,3,5-triazine,
2,4,6-tris(cycloheptadecylidene-aminooxy)-1,3,5-triazine,
2,4-dichloro-6-cyclooctadecylidene-aminooxy-1,3,5-triazine,
2-chloro-4,6-bis(cyclooctadecylidene-aminooxy)-1,3,5-triazine and
2,4,6-tris(cyclooctadecylidene-aminooxy)-1,3,5-triazine.

Among these, compounds particularly useful in preparing a laurolactam include 2,4-dichloro-6-cyclododecylidene-aminooxy-1,3,5-triazine (Compound 1) and 2-chloro-4,6-bis(cyclododecylidene-aminooxy)-1,3,5-triazine (Compound 2) represented by the following formulae. These compounds are novel.

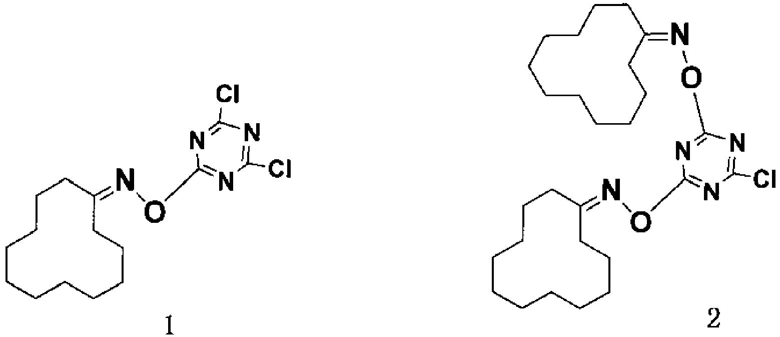

According to the present invention, the
cycloalkylidene-aminooxy-1,3,5-triazine compound is selected from the group consisting of 2,4-dichloro-6-cyclododecylidene-aminooxy-1,3,5-triazine and 2-chloro-4,6-bis(cyclododecylidene-aminooxy)-1,3,5-triazine.

The novel compounds,
2,4-dichloro-6-cyclododecylidene-aminooxy-1,3,5-triazine (Compound 1) and 2-chloro-4,6-bis(cyclododecylidene-aminooxy)-1,3,5-triazine (Compound 2) are prepared by reacting a cyclododecanone oxime (Compound 3) with a trichlorotriazine (Compound 4) in an organic solvent in the presence or absence of a base as shown in the following reaction scheme.

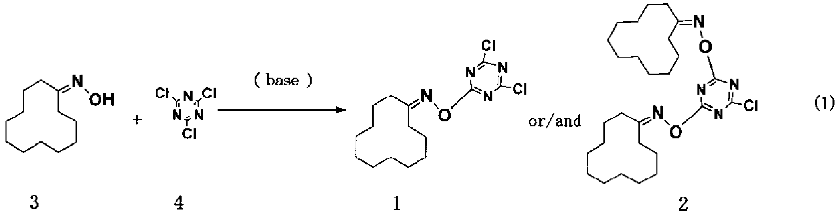

A ratio of cyclododecanone oxime to trichlorotriazine is 0.5:1 to 1.5:1, preferably 0.7:1 to 1.2:1 when preparing
2,4-dichloro-6-cyclododecylidene-aminooxy-1,3,5-triazine (Compound 1). It is 1.6:1 to 3:1, preferably 1.8:1 to 2.5:1 when preparing
2-chloro-4,6-bis(cyclododecylidene-aminooxy)-1,3,5-triazine (Compound 2).

Examples of a base may include, but not limited to, inorganic bases such as sodium hydroxide, sodium hydrogen carbonate and sodium carbonate and organic bases such as triethylamine, N,N-diisopropylethylamine, pyridine, N,N-dimethylaminopyridine and N,N-dimethylaniline, but it should be noted that when an inorganic base is less soluble in an organic solvent, the system becomes acidic, which tends to cause a side reaction. It should be further noted that when using a nucleophilic organic base such as triethylamine, it may react with trichlorotriazine, leading to addition of a diethylamino group by a substitution reaction. A particularly preferable base is N,N-diisopropylethylamine which is soluble in an organic solvent and less nucleophilic.

The amount of the base is 0 to 5 moles, preferably 0.5 to 1.5 moles to one mole of cyclododecanone oxime.

There are no particular restrictions to an organic solvent as long as it does not inhibit this reaction, and examples include toluene, benzene, xylenes, cyclohexane, cyclooctane and isopropylcyclohexane, particularly preferably toluene.

A concentration of cyclododecanone oxime is 3 to 80 % by weight, preferably 5 to 60 % by weight.

A reaction temperature is conducted preferably at a temperature of a boiling point of a solvent used or lower. It is -10 to 80 °C, preferably 0 to 60 °C when preparing
2,4-dichloro-6-cyclododecylidene-aminooxy-1,3,5-triazine (Compound 1). It is 20 °C to a boiling point of a solvent, preferably 30 °C to 110 °C when preparing 2-chloro-4,6-bis(cyclododecylidene-aminooxy)-1,3,5-triazine (Compound 2).

There are no particular restrictions to a reaction pressure and the reaction can be conducted at an ambient pressure or an increased pressure. A reaction time varies depending on the reaction conditions such as a concentration and a temperature, and it is generally 0.01 to 24 hours, preferably, 0.05 to 6 hours.

In terms of workup after the reaction, a crude product can be obtained, for example, by removing salts and the like as byproducts through silica gel and then removing a solvent.
2,4-Dichloro-6-cyclododecylidene-aminooxy-1,3,5-triazine (Compound 1) and 2-chloro-4,6-bis(cyclododecylidene-aminooxy)-1,3,5-triazine (Compound 2) obtained as a crude product can be separated and purified by, for example, an appropriate method such as column chromatography and crystallization. There are no particular restrictions to a reactor, and the reaction can be conducted in a reactor equipped with a common stirring device.

Other cycloalkylidene-aminooxy-1,3,5-triazine compounds can be produced as described for preparation of Compounds 1 and 2. A cycloalkanone oxime compound can be prepared from a cycloalkanone compound and hydroxylamine sulfate, for example, as described in Patent reference 3.

### Preparation of a lactam

A cycloalkylidene-aminooxy-1,3,5-triazine compound can be used as a catalyst in producing a lactam compound from a cycloalkanone oxime compound (Embodiment 1) or as a direct starting material for producing a lactam compound (Embodiment 2).
According to the present invention, a cycloalkylidene-aminooxy-1,3,5-triazine compound selected from the group consisting of
2,4-dichloro-6-cyclododecylidene-aminooxy-1,3,5-triazine and
2-chloro-4,6-bis(cyclododecylidene-aminooxy)-1,3,5-triazine is used as a catalyst in producing a lactam compound from a cycloalkanone oxime compound (Embodiment 1 as claimed in claim 1) or as a direct starting material for producing a lactam compound (Embodiment 2 as claimed in claim 2).

When being used as a catalyst (Embodiment 1), the above compound is used in the amount of 50 mol % or less, preferably 20 mol % or less, more preferably 10 mol % or less of the starting material of cycloalkanone oxime compound. It is generally used in the amount of 0.1 mol % or more. When being used as a catalyst, cycloalkylidene-aminooxy-1,3,5-triazine compound also produces a lactam. Accordingly, a pure lactam is obtained by using a cycloalkylidene-aminooxy-1,3,5-triazine compound corresponding to a starting cycloalkanone oxime compound. For example, when cyclododecanone oxime is used as a starting material to prepare laurolactam, the catalyst to be used is cyclododecylidene-aminooxy-1,3,5-triazine compound. Assuming that a theoretical yield from a starting cycloalkanone oxime compound is 100 %, a lactam is formed in 100 % or more.

In both Embodiments 1 and 2, the reaction is conducted in a solvent selected from the group consisting of aromatic hydrocarbons such as benzene, toluene, xylenes, cumene and chlorobenzene, and acetonitrile. Examples of other solvents include aromatic hydrocarbons such as benzene, toluene, xylenes, cumene and chlorobenzene; aliphatic hydrocarbons such as n-hexane, n-heptane, n-nonane, cyclohexane, cyclooctane, cyclodecane, cyclododecane and hydrocumene; ketones such as acetone, methyl ethyl ketone, methyl isopropyl ketone, methyl isobutyl ketone, cyclohexanone and cyclododecanone; nitriles such as acetonitrile, propionitrile and benzonitrile; amides such as N,N-dimethylformamide, N,N-dimethylacetamide, N-methylpyrrolidone and 1,3-dimethyl-2-imidazolinone; sulfoxides and sulfones such as dimethyl sulfoxide and sulfolane; esters such as ethyl formate, methyl acetate, ethyl acetate, methyl propionate and ethyl butanoate; carboxylic acid such as formic acid, acetic acid, propionic acid and butanoic acid; ethers such as diethyl ether, diisopropyl ether, tetrahydrofuran and dioxane, phosphoramides such as hexamethyl phosphortriamide; and alcohols such as methanol, ethanol, propanol and isopropanol. Preferred are aromatic hydrocarbons and nitriles, particularly toluene and acetonitrile. As the solvent, a commercially available solvent may be directly used without any particular dehydrating treatment.

In Embodiment 1, the amount of the solvent is, but not limited to, generally 0.3 to 100 parts by weight, preferably 1 to 50 parts by weight to one part of a cycloalkanone oxime compound.

In Embodiment 2, the amount of the solvent is, but not limited to, generally 0.3 to 100 parts by weight, preferably 1 to 50 parts by weight to one part of a cycloalkylidene-aminooxy-1,3,5-triazine compound.

In both Embodiments 1 and 2, the reaction can be conducted in a solvent under heating and/or in the presence of an acid for accelerating the reaction. A reaction temperature is, but not limited to, preferably in the range of 0 to 150 °C.

Examples of the acid include, but not limited to, various Broensted acids such as hydrogen chloride and methanesulfonic acid and Lewis acids such as zinc chloride and iron chlorides, particularly preferably hydrogen chloride. Its amount is 0.1 to 20 mol equivalents, preferably 0.5 to 15 mol equivalents to the cycloalkylidene-aminooxy-1,3,5-triazine compound.

There are no particular restrictions to a reaction pressure, and the reaction can be conducted at an ambient pressure or at an increased pressure. A reaction time varies depending on the reaction conditions such as a concentration and a temperature, and it is generally 0.01 to 24 hours, preferably, 0.05 to 10 hours.

The reaction may be followed by workup such as extraction to give a lactam. There are no particular restrictions to a reactor, and the reaction can be conducted in a reactor equipped with a common stirring device. The lactam prepared according to the present invention can be separated and purified by a common process such as distillation and crystallization.

### EXAMPLES

There will be specifically described the present invention with reference to Examples.

### Example 1

To 19.6 g of acetonitrile were added 199 mg (1 mmol) of cyclododecanone oxime and 17.4 mg (0.05 mmol) of
2,4-dichloro-6-cyclododecylidene-aminooxy-1,3,5-triazine. The mixture was heated with stirring at 78 °C for one hour. Quantitative analysis by HPLC indicated that laurolactam was obtained in an yield of 100 % or more on the basis of the starting cyclododecanone oxime.

### Example 2

To 19.7 g of acetonitrile were added 202 mg (1 mmol) of cyclododecanone oxime, 26.7 mg (0.05 mmol) of
2-chloro-4,6-bis(cyclododecylidene-aminooxy)-1,3,5-triazine and 2.5 mL (0.1 mmol) of 0.04 M hydrogen chloride in acetonitrile. The mixture was heated with stirring at 78 °C for one hour. Quantitative analysis by HPLC indicated that laurolactam was obtained in an yield of 100 % or more on the basis of the starting cyclododecanone oxime.

### Example 3

In 10 g of acetonitrile was dissolved 103 mg (0.30 mmol) of 2,4-dichloro-6-cyclododecylidene-aminooxy-1,3,5-triazine. The mixture was heated with stirring at 77 °C for 5 hours. Quantitative analysis by HPLC indicated that 56 mg (0.28 mmol) of laurolactam was obtained.

### Example 4 (not according to the present invention)

In 10 g of acetonitrile was dissolved 201 mg (0.40 mmol) of 2-chloro-4,6-bis(cyclododecylidene-aminooxy)-1,3,5-triazine and 1.5 g of a 4M solution of hydrogen chloride in dioxane. After stirring several minutes at room temperature, quantitative analysis by HPLC indicated that 159 mg (0.80 mmol) of laurolactam was obtained.

### Reference Example 1: Synthesis of 2,4-dichloro-6-cyclododecylidene-aminooxy-1,3,5-triazine (Compound 1)

In 120 mL of toluene was dissolved 3 g (15 mmol) of cyclododecanone oxime, and under water cooling, 3 g (16 mmol) of trichlorotriazine was added. Then, to the mixture was slowly added dropwise 15 mL of a solution of N,N-diisopropylethylamine in toluene (toluene was added to 2.0 g (15 mmol) of N,N-diisopropylethylamine to 15 mL). After stirring at room temperature for 1.5 hours, the reaction solution was filtrated by suction through silica gel. The silica gel was washed with a 20:1 solution of toluene-ethyl acetate. The filtrate was evaporated in vacuo to obtain a pale yellow solid, which was then recrystallized from hexane to give 4.6 g of the desired product as colorless prism crystals.
m.p. 118 to 120 °C;
¹H-NMR (δ ppm, CDCl₃); 1.15 to 1.81 (18H, m), 2.48 to 2.61 (4H, m);
Elementary analysis;
observed: H: 6.31, C: 52.14, N: 16.15, O: 5.04;
calculated for C₁₅H₂₂Cl₂N₄O: H: 6.42, C: 52.18, N: 16.23, O: 4.63.

### Reference Example 2: Synthesis of 2-chloro-4,6-bis(cyclododecylidene-aminooxy)-1,3,5-triazine (Compound 2)

In 160 mL of toluene was dissolved 4 g (20 mmol) of cyclododecanone oxime, and under water cooling, 1.9 g (10 mmol) of trichlorotriazine was added. Then, to the mixture was slowly added dropwise 15 mL of a solution of N,N-diisopropylethylamine in toluene (toluene was added to 2.66 g (21 mmol) of N,N-diisopropylethylamine to 15 mL). After stirring at 68 °C for 3 hours, the reaction solution was filtrated by suction through silica gel. The silica gel was washed with a 10:1 solution of toluene-ethyl acetate. The filtrate was evaporated in vacuo to obtain a residue, which was then purified by column chromatography (Wako Gel C-200, toluene:ethyl acetate = 20:1). The crude crystals thus obtained were recrystallized from hexane to give 1.8 g of the desired product as colorless needle crystals.
m.p.: 113 to 114 °C;
¹H-NMR (δ ppm, CDCl₃) 1.26 to 1.80 (36H, m), 2.35 to 2.61 (8H, m);
Elementary analysis;
observed: H: 8.55, C: 64.08, N: 13.73, O: 6.58;
calculated for C₂₇H₄₄ClN₅O₂: H: 8.76, C: 64.07, N: 13.84, O: 6.32.

### Reference Example 3: Investigation for synthesizing a cyclododecylidene-aminooxy-1,3,5-triazine compound using triethylamine as a base(investigation in accordance with the method described in Non-patent reference 1)

To 150 mL of anhydrous acetonitrile was added 2 g (10 mmol) of cyclododecanone oxime, which was, however, not dissolved, and therefore, 50 mL of toluene was added to dissolve the solid. To the solution were slowly added 0.62 g (3.4 mmol) of trichlorotriazine and 1.1 g (11 mmol) of triethylamine in sequence. After stirring at room temperature for one hour, the mixture was allowed to stand for 3 days and then evaporated in vacuo. After adding toluene, the insoluble matter was filtered off and the filtrate was evaporated under a reduced pressure. The residue was purified by column chromatography (Wako Gel C-200, toluene:ethyl acetate = 5:1). The crude crystals thus obtained were recrystallized from hexane to give 0.55 g of colorless columnar crystals. Analysis of this compound indicated that it was 2-(N,N-diethylamino)-4,6-bis(cyclododecylidene-aminooxy)-1,3,5-triazine. The desired tris(cyclododecylidene-aminooxy)-1,3,5-triazine, 2,4-dichloro-6-cyclododecylidene-aminooxy-1,3,5-triazine (Compound 1) or 2-chloro-4,6-bis(cyclododecylidene-aminooxy)-1,3,5-triazine (Compound 2) was not obtained.

## Claims

1. A process for producing a lactam compound from a cycloalkanone oxime compound in a reaction process in the presence of:
(a) a cycloalkylidene-aminooxy-1,3,5-triazine compound selected from the group consisting of 2,4-dichloro-6-cyclododecylidene-aminooxy-1,3,5-triazine and 2-chloro-4,6-bis(cyclododecylidene-aminooxy)-1,3,5-triazine as a Beckmann rearrangement catalyst,
(b) a solvent selected from the group consisting of aromatic hydrocarbons and acetonitrile, and
(c) optionally, an acid;
wherein said lactam compound is laurolactam.

2. A process for producing a lactam compound, wherein a cycloalkylidene-aminooxy-1,3,5-triazine compound selected from the group consisting of 2,4-dichloro-6-cyclododecylidene-aminooxy-1,3,5-triazine and 2-chloro-4,6-bis(cyclododecylidene-aminooxy)-1,3,5-triazine is provided as a reaction starting material in a reaction using a solvent selected from the group consisting of aromatic hydrocarbons and acetonitrile, and wherein said lactam compound is laurolactam.

3. A Beckmann rearrangement catalyst selected from the group consisting of 2,4-dichloro-6-cyclododecylidene-aminooxy-1,3,5-triazine and 2-chloro-4,6-bis(cyclododecylidene-aminooxy)-1,3,5-triazine, used in a reaction process to prepare a lactam compound from a cycloalkanone oxime compound in the presence of:
(a) a solvent selected from the group consisting of aromatic hydrocarbons and acetonitrile, and
(b) optionally, an acid;
wherein said lactam compound is laurolactam.

4. 2,4-Dichloro-6-cyclododecylidene-aminooxy-1,3,5-triazine or 2-chloro-4,6-bis(cyclododecylidene-aminooxy)-1,3,5-triazine.

## Patentansprüche

1. Verfahren zur Herstellung einer Lactamverbindung aus einer Cycloalkanonoxim-Verbindung in einem Reaktionsverfahren in Gegenwart von:
(a) einer Cycloalkyliden-aminooxy-1,3,5-triazin-Verbindung ausgewählt aus der Gruppe bestehend aus 2,4-Dichlor-6-cyclododecyliden-aminooxy-1,3,5-triazin und 2 - Chlor-4,6-bis (cyclododecyliden-aminooxy)-1,3,5-triazin als ein Beckmann-Umlagerungskatalysator
(b) einem Lösungsmittel ausgewählt aus der Gruppe bestehend aus aromatischen Kohlenwasserstoffen und Acetonitril und
(c) gegebenenfalls einer Säure;
wobei die Lactamverbindung Laurinlactam ist.

2. Verfahren zur Herstellung einer Lactamverbindung, wobei eine Cycloalkyliden-aminooxy-1,3,5-triazin-Verbindung ausgewählt aus der Gruppe bestehend aus 2,4-Dichlor-6-cyclododecyliden-aminooxy-1,3,5-triazin und 2-Chlor-4,6-bis-(cyclododecyliden-aminooxy)-1,3,5-triazin als ein Reaktionsausgangsmaterial in einer Reaktion unter Verwendung eines Lösungsmittels ausgewählt aus der Gruppe bestehend aus aromatischen Kohlenwasserstoffen und Acetonitril bereitgestellt wird, und wobei die Lactamverbindung Laurinlactam ist.

3. Beckmann-Umlagerungskatalysator ausgewählt aus der Gruppe bestehend aus 2,4-Dichlor-6-cyclododecyliden-aminooxy-1,3,5-triazin und 2-Chlor-4,6-bis-(cyclododecylidenaminooxy)-1,3,5-Triazin, der in einem Reaktionsverfahren zur Herstellung einer Lactamverbindung aus einer Cycloalkanon-oxim-Verbindung in Gegenwart von:
(a) einem Lösungsmittel ausgewählt aus der Gruppe bestehend aus aromatischen Kohlenwasserstoffen und Acetonitril und
(b) gegebenenfalls einer Säure;
verwendet wird,
wobei die Lactamverbindung Laurinlactam ist.

4. 2,4-Dichlor-6-cyclododecyliden-aminooxy-1,3,5-triazin oder 2-Chlor-4,6-bis-(cyclododecylidenaminooxy)-1,3,5-triazin.

## Revendications

1. Procédé de production d'un composé lactame à partir d'un composé cycloalcanone oxime dans un processus de réaction en présence de :
(a) un composé cycloalkylidène-aminooxy-1,3,5-triazine sélectionné dans le groupe constitué de 2,4-dichloro-6-cyclododécylidène-aminooxy-1,3,5-triazine et 2-chloro-4,6-bis(cyclododécylidène-aminooxy)-1,3,5-triazine en tant que catalyseur de réarrangement de Beckmann,
(b) un solvant sélectionné dans le groupe constitué d'hydrocarbures aromatiques et d'acétonitrile, et
(c) optionnellement, un acide ;
dans lequel ledit composé lactame est le laurolactame.

2. Procédé de production d'un composé lactame, dans lequel un composé cycloalkylidène-aminooxy-1,3,5-triazine sélectionné dans le groupe constitué de la 2,4-dichloro-6-cyclododécylidène-aminooxy-1,3,5-triazine et de la 2-chloro-4,6-bis(cyclododécylidène-aminooxy)-1,3,5-triazine est fourni en tant que matière première de réaction dans une réaction utilisant un solvant sélectionné dans le groupe constitué des hydrocarbures aromatiques et de l'acétonitrile, et dans lequel ledit composé lactame est le laurolactame.

3. Catalyseur de réarrangement de Beckmann sélectionné dans le groupe constitué de la 2,4-dichloro-6-cyclododécylidène-aminooxy-1,3,5-triazine et de la 2-chloro-4,6-bis(cyclododécylidène-aminooxy)-1,3,5-triazine, utilisé dans un processus de réaction pour préparer un composé lactame à partir d'un composé cycloalcanone oxime en présence de :
(a) un solvant sélectionné dans le groupe constitué des hydrocarbures aromatiques et de l'acénonitrile, et
(b) optionnellement, un acide ;
dans lequel ledit composé lactame est le laurolactame.

4. 2,4-dichloro-6-cyclododécylidène-aminooxy-1,3,5-triazine ou 2-chloro-4,6-bis(cyclododécylidène-aminooxy)-1,3,5-triazine.
